Europäisches Patentamt

European Patent Office

Office européen des brevets

(11) Veröffentlichungsnummer: **0 459 402 A2**

# EUROPÄISCHE PATENTANMELDUNG

(21) Anmeldenummer: 91108702.1

(22) Anmeldetag: 28.05.91

(51) Int. Cl.5: **A61N 2/04**

(30) Priorität: 29.05.90 DE 9006057 U

(43) Veröffentlichungstag der Anmeldung:
04.12.91 Patentblatt 91/49

(84) Benannte Vertragsstaaten:
**AT CH DE ES FR GB IT LI SE**

(71) Anmelder: **Kraus, Werner, Dipl.-Ing.**
**Augustenstrasse 41**
**W-8000 München 2(DE)**

(72) Erfinder: **Kraus, Werner, Dipl.-Ing.**
**Augustenstrasse 41**
**W-8000 München 2(DE)**

(74) Vertreter: **von Bezold, Dieter, Dr. et al**
**Dr. Dieter von Bezold Dipl.-Ing. Peter Schütz**
**Dipl.-Ing. Wolfgang Heusler Brienner Strasse 52**
**W-8000 München 2(DE)**

(54) **Elektromedizinisches Gerät zum Erzeugen niederfrequenter Magnetfelder.**

(57) Ein elektromedizinisches Gerät zum Erzeugen niederfrequenter Magnetfelder für die Magnetfeldtherapie oder Behandlungen nach der Methode von Kraus und Lechner enthält eine Einrichtung (10, 12) zum Erzeugen niederfrequenter elektrischer Schwingungen einstellbarer Frequenz und Amplitude, und mindestens eine Applikatorspule (32) zum Erzeugen eines niederfrequenten Magnetfeldes entsprechend der elektrischen Schwingungen. Das Einschalten des Gerätes und das Einleiten eines Behandlungszyklus erfolgt durch Betätigen eines einzigen Tastenschalters (56, 58). Das Gerät wird normalerweise nach Ablauf einer Behandlungsperiode durch eine Zeitschaltvorrichtung 80 abgeschaltet, es kann jedoch auch durch eine Fehlererkennungsschaltung (88) abgeschlatet werden, die beim Auftreten von Störungen das Gerät automatisch abschaltet. Durch diese Maßnahme werden Bedienungsfehler und Beschädigungen des Gerätes durch Störungen vermieden, auch wenn das Gerät von Laien zu Hause ohne Aufsicht benutzt wird.

Fig. 1

EP 0 459 402 A2

Die vorliegende Erfindung betrifft ein elektromedizinisches Gerät mit den im Oberbegriff des Patentanspruchs 1 angegebenen Merkmalen.

Geräte der obengenannten Art sind unter der Bezeichnung "Magnetodyn$^{(Wz)}$-Geräte" bekannt und dienen zum Erzeugen von im wesentlichen sinusförmigen Magnetfeldern mit Frequenzen zwischen etwa 2 und 30 Hz und Feldstärken bis zu etwa 5mT. Die Magnetfelder können entweder selbst zur Behandlung dienen, z. B. bei Lockerung von Gelenk-Endoprothesen, verzögerter Heilung von Knochendefekten, gestörter Wundheilung u. a. m. oder zur Induktion niederfrequenter Wechselströme in implantierten Aufnehmerspulen zur Beschleunigung der Heilung von Knochenbrüchen, Pseudoarthrosen u. a. m. gemäß dem Verfahren nach Kraus und Lechner verwendet werden.

Um die Arztpraxen und Krankenhäuser zu entlasten werden die elektromedizinischen Geräte der hier interessierenden Art in zunehmendem Umfang für die Dauer der Behandlung an die Patienten vermietet, so daß die Behandlung zu Hause erfolgen kann. Dies bringt jedoch gewisse Probleme mit sich, da die Geräte dann normalerweise von einem Laien ohne Hilfe und Überwachung benutzt werden müssen. Die Bedienung muß daher einfach sein und unabsichtliche oder absichtliche Manipulationen und Fehlbedienungen müssen ausgeschlossen werden. Außerdem ist eine selbsttätige Sicherung gegen Fehlfunktionen erwünscht, da diese der Laie normalerweise nicht erkennen kann.

Die vorliegende Erfindung löst dementsprechend die Aufgabe, ein elektromedizinisches Gerät zum Erzeugen niederfrequenter Magnetfelder anzugeben, welches einfach, sicher und zuverlässig im Betrieb ist, so daß es auch von Laien problemlos benutzt werden kann.

Im folgenden wird ein bevorzugtes Ausführungsbeispiel eines elektromedizinischen Gerätes gemäß der Erfindung unter Bezugnahme auf die Zeichnungen näher erläutert. Es zeigen:

Fig. 1    ein vereinfachtes Schaltbild eines Gerätes gemäß einer Ausführungsform der Erfindung;

Fig. 2    eine Frontansicht einer praktischen Ausführungsform eines Gerätes gemäß Fig. 1 und

Fig. 3    eine Ansicht der Rückseite des Gerätes gemäß Fig. 2.

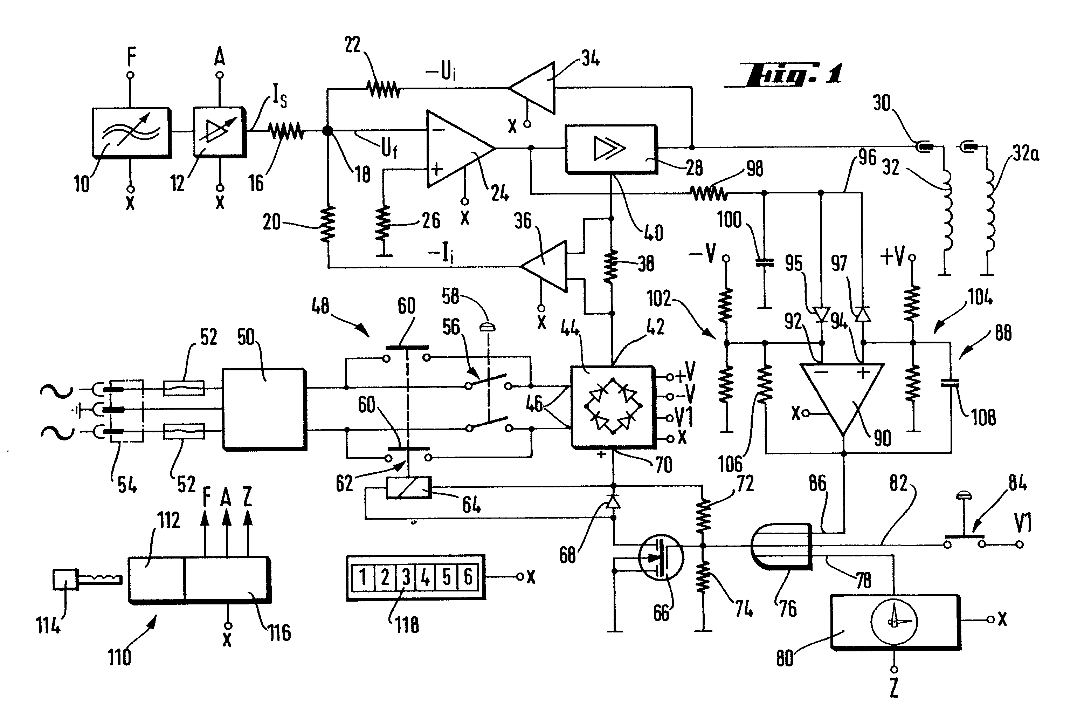

Das in Fig. 1 dargestellte Gerät enthält eine Einrichtung zum Erzeugen frequenzveränderlicher elektrischer Schwingungen in Form eines Sinusoszillators, dessen Frequenz bei dem vorliegenden Ausführungsbeispiel durch ein Signal F auf einen gewünschten Wert zwischen 2 und 30 Hz einstellbar ist. Die Sinusschwingung vom Ausgang des Oszillators 10 wird einer Verstärkerschaltung 12 zugeführt, deren Verstärkungsgrad zur Einstellung der Amplitude der Sinusschwingungen durch ein Signal A verstellbar ist. Das Ausgangssignal der Verstärkerschaltung 12 stellt ein Stromsollwertsignal $I_s$ dar, welches über einen Widerstand 16 einem Summenpunkt 18 zugeführt wird. Dem Summenpunkt 18 werden ferner ein negatives Strom-Istwertsignal $-I_i$ über einen Widerstand 20 sowie ein negatives Spannungs-Istwertsignal $-U_i$ über einen Widerstand 22 zugeführt. Am Summenpunkt 18 entsteht dadurch ein Regel- oder Fehlersignal $U_f$, das dem Minus-Eingang eines Differenzverstärkers 24 zugeführt wird, dessen Plus-Eingang über einen Widerstand 26 an Masse liegt. Das Ausgangssignal des Differenzverstärkers 24 wird dem Eingang einer Leistungs- und Endverstärkerschaltung 28 zugeführt, deren Ausgang mit einem Anschluß 30 für eine Applikatorspule 32 gekoppelt ist. Vom Ausgang der Verstärkerschaltung 28 wird außerdem ein der Spannung an der Applikatorspule entsprechendes Signal abgegriffen und über einen Trennverstärker 34 sowie den Widerstand 22 dem Summenpunkt 18 als Spannungs-Istwertsignal zugeführt.

Das Strom-Istwertsignal wird durch einen Differenzverstärker 36 erzeugt, zwischen dessen Eingänge ein Stromabgreifwiderstand 38 kleinen Widerstandwertes geschaltet ist und dessen Ausgang über den Widerstand 20 mit dem Summenpunkt 18 gekoppelt ist. Der Stromabgreifwiderstand ist zwischen einen Betriebsspannungseingang 40 der Verstärkerschaltung 28 und einen entsprechenden Ausgang 42 eines Netzteiles 44 geschaltet, welches einen Netztransformator sowie Gleichrichterschaltungen enthält und in üblicher Weise die für den Betrieb der Schaltungsanordnungen des Gerätes erforderlichen Betriebs- und Vorspannungen liefert.

Das Netzteil 44 ist mit seinen Netzspannungseingangsanschlüssen 46 über eine Schaltvorrichtung 48, ein Entstörungsfilter 50 und Sicherungen 52 mit einem Netzstecker 54 gekoppelt.

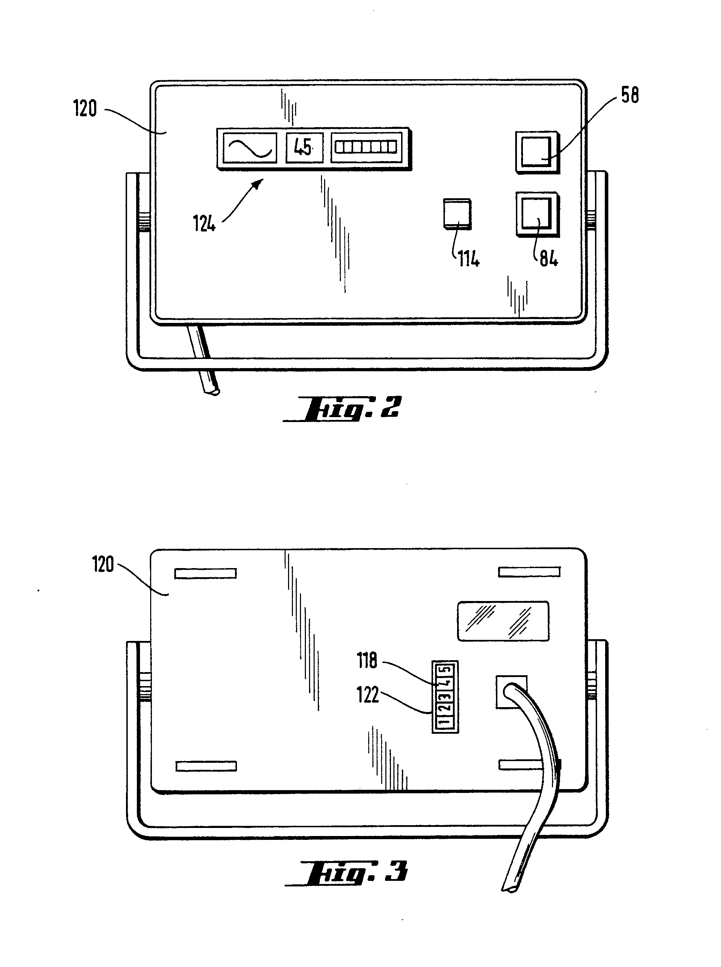

Die Schaltvorrichtung enthält einen zweipoligen Tastenschalter 56, der durch eine nicht dargestellte Feder in Öffnungsrichtung vorgespannt ist und das Netzgerät 44 so lange mit dem Netz verbindet, wie eine an der Frontplatte des Gerätes (Fig. 2) angeordnete Drucktaste 58 gedrückt wird. Den Kontaktsätzen des Tastenschalters 56 sind Arbeitskontakte 60 eines elektromagnetischen Relais 62, das eine Wicklung 64 hat, parallelgeschaltet.

Die Wicklung 64 ist zwischen eine Betriebsspannungsklemme 70 des Netzgerätes 44 und die Drainelektrode eines Feldeffekttransistors 66 geschaltet, dessen Sourceelektrode an Masse liegt. Die Wicklung 64 ist mit einer Schutzdiode 68 überbrückt. Das Gate des Feldeffekttransistors 66 erhält eine Fluß-Vorspannung vom Verbindungspunkt zwischen zwei Widerständen 72 und 74, die in Reihe zwischen die Betriebsspannungsklemme 70 und Masse geschaltet sind. Das Gate des Feldeffekttransistors 66 ist außerdem mit dem Ausgang eines ODER-Gliedes 76 verbunden, das bei Erregung mindestens eines seiner Eingänge eine Ausgangsspannung liefert, welche den Feldeffekttransistor 66 sperrt, wodurch die Relaiswicklung 64 stromlos wird. Ein erster Eingang 78 des ODER-Gliedes ist mit einer Zeitsteuereinrichtung 80 verbunden, die bei Einschalten des Gerätes mittels der Schaltvorrichtung 48 vom Netzgerät 44 eine Betriebsspannung x erhält, dann zu laufen beginnt und nach einer vorgegebenen Zeitspanne (Dauer eines Behandlungszyklus), die durch ein Signal Z bestimmt wird, ein Ausgangssignal an den Eingang 78 des ODER-Gliedes 76 liefert, welches den Feldeffekttransistor 66 sperrt und damit das Relais 72 zum Abfallen bringt, so daß das Gerät ausgeschaltet wird.

Ein zweiter Eingang 82 des ODER-Gliedes ist über einen Tastenschalter 84, der das Gerät augenblicklich abzuschalten gestattet, mit einem Vorspannungsanschluß V1 des Netzgerätes 44 verbunden. Beim Drücken des Tastenschalters 84 wird der Feldeffekttransistor 66 ebenfalls gesperrt, so daß das Relais 62 abfällt.

Ein dritter Eingang 86 des ODER-Gliedes 76 ist mit einer Fehlererkennungsschaltung 88 verbunden, welche beim Auftreten eines Fehlers im Gerät ein Abschaltsignal an das ODER-Glied 76 liefert. Die Fehlererkennungsschaltung 88 spricht an, wenn in der normalerweise sinusförmigen Ausgangsspannung des in der Regelschleife liegenden Differenzverstärkers 24 Unsymmetrien oder übermäßig hohe Signalamplituden, die einen vorgegebenen Schwellenwert in positiver oder negativer Richtung überschreiten, auftreten. Die Fehlererkennungsschaltung 88 enthält einen Differenzverstärker 90, dessen Ausgang mit dem Eingang 86 des ODER-Gliedes 76 gekoppelt ist und dessen Eingänge 92, 94 über jeweils eine Diode 95, 97, die entgegengesetzt gepolt sind, mit einer Leitung 96 verbunden sind, die über einen Widerstand 98 an den Ausgang des Differenzverstärkers 24 angeschlossen ist. Zwischen die Leitung 96 und Masse ist ein Kondensator 100 geschaltet, der verhindert, daß die Fehlererkennungsschaltung schon bei kurzzeitigen Störsignalen anspricht. Die Eingänge 92, 94 des Differenzverstärkers 90 sind jeweils mit einer Vorspannungsschaltung 102 bzw. 104 verbunden. Der Minus-Eingang 92 ist außerdem über einen Gegenkopplungswiderstand 106 mit dem Ausgang des Differenzverstärkers 90 verbunden. Zwischen den Plus-Eingang 94 und den Ausgang ist ein Kondensator 108 geschaltet. Die Fehlererkennungsschaltung 88 liefert so lange kein Ausgangssignal wie die sinusförmige Ausgangsspannung des Differenzverstärkers 24 nullsymmetrisch ist und weder in positiver noch in negativer Richtung entsprechende Schwellenwerte überschreitet. Sobald jedoch ein Fehler auftritt, der ein übermäßiges Auswandern der Spannung am Ausgang des Differenzverstärkers 24 zur Folge hat, erzeugt der Differenzverstärker 90 ein Ausgangssignal, welches das Relais 62 zum Abfallen bringt und das Gerät dadurch abschaltet.

Alle Schaltungen und Einrichtungen des Gerätes erhalten Betriebsspannungen x und Vorspannungen dann und nur dann, wenn das Netzgerät 44 an Netzspannung liegt. Um einen Behandlungszyklus einzuleiten, braucht also nur die Drucktaste 58 gedrückt zu werden und nach Ablauf der vorgegebenen Behandlungsdauer wird das Gerät durch die Zeitsteuereinrichtung 80 automatisch abgeschaltet. Der früher häufig vorkommende Fehler, daß entweder nur der Netzschalter oder nur der zur Einleitung des Behandlungsbeginnes dienende Startschalter betätigt wurden und das Gerät daher nicht eingeschaltet wurde, können bei dem vorliegenden Gerät nicht auftreten.

Eine weitere Sicherung gegen Fehlbedienungen wird dadurch erreicht, daß die Behandlungsparameter, nämlich Frequenz, Amplitude und Dauer der Behandlung nicht mehr vom Patienten willkürlich eingestellt werden können, sondern durch eine Codiereinheit 110 bestimmt werden. Die Codiereinheit enthält einen Codeschalter 112, welcher durch Einstecken eines Codierschlüssels 114 einstellbar ist und Adressensignale an einen Lesespeicher (ROM) 116 liefert, welches die Signale F, A und Z, die die Frequenz, die Amplitude bzw. die Dauer eines Behandlungszyklus bestimmen, entsprechend dem Code des Codierschlüssels 114 liefert.

Die Regelschaltung mit den Komponenten 16, 18, 20, 22, 24, 26 ist für eine 80%-igen Stromregelung in Kombination mit einer 20%-igen Spannungsregelung ausgelegt. Hierdurch ist ein regelschwingungsfreies Arbeiten im ganzen Frequenzbereich gewährleistet. Die Regelung gewährleistet, daß die durch das Amplitudensignal A bestimmte magnetische Feldstärke weitestgehend frequenzunabhängig und unabhängig vom Typ der angeschlossenen Applikatorspule ist. Die Applikatorspulen 32, 32a usw. sind hierfür hinsichtlich ihres Querschnitts und ihrer Windungszahl so ausgelegt, daß sie bei einem vorgegebenen Strom alle die gleiche magnetische Feldstärke liefern.

Um die Gesamtbehandlungsdauer feststellen zu können, ist ein Betriebszeitzähler 118 vorgesehen, wie die übrigen Einheiten, eine Betriebsspannung x vom Netzteil 44 erhält, solange das Gerät eingeschaltet ist. Der Gesamtbetriebszeitzähler 118 ist im Inneren des Gehäuses 120 (Fig. 3) des Gerätes untergebracht und kann durch einen unauffälligen Schlitz 122, der sich an der Rückseite des Gerätes findet, abgelesen werden.

Die Frontplatte des Geräts (Fig. 2) enthält ein Anzeigefeld 124 zur Anzeige der Frequenz, der noch verbleibenden Dauer des augenblicklichen Behandlungszyklus und der Strom- bzw. Magnetfeldamplitude. Die dem Anzeigefeld zugeordneten Schaltungen sind in üblicher Weise ausgebildet und daher nicht dargestellt.

**Patentansprüche**

1. Elektromedizinisches Gerät zum Erzeugen niederfrequenter Magnetfelder mit einem Generatorteil, welcher

   a) eine Einrichtung (10) zum Erzeugen niederfrequenter elektrischer Schwingungen,
   b) eine Einrichtung (110) zum Einstellen der Frequenz der Schwingungen,
   c) eine Einrichtung (12) zum Einstellen der Amplitude der Schwingungen,
   d) eine Zeitsteuereinrichtung (80) zum Einstellen der Dauer der Schwingungserzeugung,
   e) ein Netzteil (44) zum Erzeugen von Betriebs- und Vorspannungen für die genannten Einrichtungen und
   f) einen Netzschalter (62) zum Verbinden des Netzteiles (44) mit einem Netzanschluß (54)

   enthält und mit mindestens einer an den Generatorteil anschließbaren, durch die niederfrequenten elektrischen Schwingungen gespeisten Applikatorspule (32) zum Erzeugen eines den elektrischen Schwingungen entsprechenden niederfrequenten Magnetfeldes, **dadurch gekennzeichnet,** daß der Netzschalter (62) einen Tastenschalter (56, 58), der zwischen den Netzanschluß (54) und das Netzteil (44) geschaltet ist und sie verbindet, solange er gedrückt wird, und ein Relais (62) enthält, welches dem Tastschalter (56) parallelgeschaltete Arbeitskontakte (60) und eine durch das Netzteil (44) gespeiste Wicklung hat und daß die Betriebs- und Vorspannungen den genannten Einrichtungen direkt, ohne Zwischenschaltung einer weiteren Schaltvorrichtung zugeführt sind, so daß das Gerät beim Einschalten des Netzschalters zu arbeiten beginnt.

2. Gerät nach Anspruch 1, **dadurch gekennzeichnet,** daß die elektrischen Schwingungen einstellbarer Frequenz und Amplitude als Sollwertsignal ($I_s$) einer Regelschaltung (16, 18, 20, 22, 24, 26) zugeführt ist, der außerdem ein Strom-Istwertsignal ($-I_i$) sowie ein Spannungs-Istwertsignal ($-U_i$) zugeführt sind, welche so bemessen sind, daß die Regelung in erster Linie als Stromregelung und in zweiter Linie als Spannungsregelung arbeitet.

3. Gerät nach Anspruch 2, **dadurch gekennzeichnet,** daß die Regelung eine etwa 80%-ige Stromregelung und etwa 20%-ige Spannungsregelung ist.

4. Gerät nach Anspruch 1, 2 oder 3, **gekennzeichnet durch** eine Fehlererkennungsschaltung (88), der ein Signal aus einer Regelschleife zugeführt ist und die bei Auftreten einer übermäßigen Signalamplitude ein Abschaltsignal liefert, welches das Relais (62) zum Abfallen bringt.

5. Gerät nach einem der vorhergehenden Ansprüche, **dadurch gekennzeichnet,** daß mehrere Applikatorspulen (32, 32a) unterschiedlicher Konfiguration vorgesehen sind, die wahlweise an den Generatorteil anschließbar und hinsichtlich Querschnitt sowie Windungszahl so bemessen sind, daß sie bei Speisung mit einem vorgegebenen Strom alle im wesentlichen die gleiche magnetische Feldstärke erzeugen.

6. Gerät nach einem der vorhergehenden Ansprüche, **dadurch gekennzeichnet,** daß mit dem Netzteil (44) ein Gesamtbetriebszeitzähler (118) gekoppelt ist, daß der Gesamtbetriebszeitzähler (118) im Gehäuse (120) des Gerätes angeordnet und durch einen Schlitz (122) ablesbar ist, der an einer unauffälligen Stelle des Gehäuses, wie der Rückwand, angeordnet ist.

7. Gerät nach einem der vorhergehenden Ansprüche, **dadurch gekennzeichnet,** daß die Betriebsparameter (F, A, Z) des Geräts durch eine Codiereinheit (110) wählbar sind, die ihrerseits durch einen Codierschlüssel (114) einstellbar ist.

**Fig. 1**

Fig. 2

Fig. 3